# EUROPÄISCHE PATENTSCHRIFT

(11) **EP 3 884 903 B1**
(45) Veröffentlichungstag und Bekanntmachung des Hinweises auf die Patenterteilung: **11.06.2025**
(21) Anmeldenummer: 21160304.8
(22) Anmeldetag: 02.03.2021
(51) Int. Cl.: A61F 2/00

(54) **VERPACKUNG FÜR EIN MEDIZINPRODUKT, VERFAHREN ZUR HERSTELLUNG EINER VERPACKUNG FÜR EIN MEDIZINPRODUKT, VERWENDUNG EINER VERPACKUNG ZUM VERPACKEN EINES MEDIZINPRODUKTS UND MEDIZINISCHES KIT**
PACKAGE FOR A MEDICAL PRODUCT, METHOD OF MANUFACTURING A PACKAGE FOR A MEDICAL PRODUCT, USE OF A PACKAGE FOR PACKAGING A MEDICAL PRODUCT AND MEDICAL KIT
EMBALLAGE POUR UN DISPOSITIF MÉDICAL, PROCÉDÉ DE FABRICATION D'UN EMBALLAGE POUR DISPOSITIF MÉDICAL, UTILISATION D'UN EMBALLAGE POUR EMBALLER UN DISPOSITIF MÉDICAL ET ENSEMBLE MÉDICAL

(30) Priorität: 25.03.2020 DE 102020203843
(43) Veröffentlichungstag der Anmeldung: 29.09.2021
(73) Patentinhaber: Aesculap AG, 78532 Tuttlingen (DE)
(72) Erfinder: Burmeister, Christoph, 78224 Singen (DE); Kammerer, Selina, 78083 Dauchingen (DE)
(74) Vertreter: Patentanwälte Ruff, Wilhelm, Beier, Dauster & Partner mbB

(56) Entgegenhaltungen:
- EP-A1- 2 258 323
- EP-B1- 1 729 607
- EP-B1- 2 026 675
- DE-A1- 3 917 202

## Beschreibung

### ANWENDUNGSGEBIET UND STAND DER TECHNIK

Die Erfindung betrifft eine Verpackung für ein Implantat, ein Verfahren zur Herstellung einer Verpackung für ein Implantat, die Verwendung einer Verpackung zum Verpacken eines Implantats sowie ein medizinisches Kit. Bei dem Implantat handelt es sich vorzugsweise um ein Tibia-Implantat.

Medizinprodukte können in Folge ihres Fertigungsprozesses, beispielsweise durch die Verwendung von partikelhaltigen Verpackungsmaterialien und/oder durch Reibung an ihrer Verpackung mit Partikeln belastet sein. So kann insbesondere beim Transport von Medizinprodukten ein reibungsbedingter Abrieb zwischen Produkt und Verpackungsmaterial auftreten. Handelt es sich bei dem Medizinprodukt um ein Implantat, können hierdurch Partikel (dauerhaft) in Kontakt mit Blut und/oder Geweben eines Patienten gelangen. Dadurch steigt das Risiko von schwerwiegenden postoperativen Beschwerden. Im Falle eines Tibia-Implantats kann eine solche in vivo Partikelkontamination beispielsweise zu einer Lockerung des Implantats führen.

Aus der DE 39 17 202 A1 ist ein Verpackungsbehälter bekannt, der aus einer Wanne und einem Deckel besteht, die beide miteinander zugekehrt liegenden, elastisch nachgebenden, zwischen sich medizinischen Instrumenten, Geräte, deren Teile und Implantate aufnehmenden Stützen versehen sind, wobei die Stützen aus einer großen Anzahl von über die Wannen- und Deckelbodenfläche verteilten Borsten aus elastischem Material bestehen.

Die EP 2 258 323 A1 betrifft eine Applikationsvorrichtung für Stents, die mindestens auf einem Teil der nach außen gewandten Oberfläche des Bereichs der Vorrichtung haarähnliche Fortsätze aufweist, der zum Anbringen oder Crimpen des zu applizierenden Stents vorgesehen ist.

### AUFGABE UND LÖSUNG

Der vorliegenden Erfindung liegt daher die Aufgabe zugrunde, eine Verpackung für ein Implantat bereitzustellen, welche die vorstehend im Zusammenhang von gattungsgemäßen Verpackungen genannten Nachteile weitgehend oder vollständig vermeidet. Insbesondere soll die Verpackung einen transportbedingten Abrieb zwischen Verpackung und einem optional in der Verpackung enthaltenen Implantat vermeiden oder wenigstens reduzieren. Ferner liegt der Erfindung die Aufgabe zugrunde, ein Herstellungsverfahren für eine solche Verpackung, die Verwendung einer solchen Verpackung zum Verpacken eines Implantats sowie ein entsprechendes medizinisches Kit bereitzustellen.

Diese Aufgaben werden gelöst durch eine Verpackung mit den Merkmalen gemäß unabhängigem Anspruch 1, durch ein Verfahren gemäß Anspruch 13, durch eine Verwendung gemäß Anspruch 14 sowie durch ein medizinisches Kit gemäß Anspruch 15. Bevorzugte Ausgestaltungen sind in den abhängigen Ansprüchen definiert. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung eine Verpackung für ein Implantat, wie beispielsweise ein Tibia-Implantat.

Die Verpackung zeichnet sich besonders dadurch aus, dass eine Innenoberfläche der Verpackung wenigstens abschnittsweise, insbesondere nur abschnittsweise oder durchgehend (d.h. vollflächig), mikrostrukturierte Haftelemente, insbesondere abstehende (d.h. von der Innenoberfläche der Verpackung abstehende), mikrostrukturierte Haftelemente, zum Anhaften oder Fixieren des Implantats aufweist.

Unter dem Ausdruck "Haftelemente" sollen im Sinne der vorliegenden Erfindung Strukturen verstanden werden, welche derart gestaltet sind, dass sie ein Anhaften oder Fixieren eines in der Verpackung optional enthaltenen Implantats an eine Innenoberfläche der Verpackung, vorzugsweise überwiegend oder ausschließlich auf Basis von Van-der-Waals-Kräften, ermöglichen.

Unter dem Ausdruck "mikrostrukturierte Haftelemente" sollen im Sinne der vorliegenden Erfindung Haftelemente verstanden werden, welche wenigstens eine Abmessung, wie beispielsweise Länge und/oder Durchmesser, im Mikrometerbereich, insbesondere in einem Bereich oder in einer Größenordnung von 10 µm bis 150 µm, insbesondere 50 µm bis 120 µm, bevorzugt 80 µm bis 100 µm, aufweisen. Vorzugsweise weisen die mikrostrukturierten Haftelemente im Sinne der vorliegenden Erfindung ausschließlich Abmessungen in dem vorgenannten Bereich oder in der vorgenannten Größenordnung auf.

Unter dem Ausdruck "Van-der-Waals-Kräfte" sollen im Sinne der vorliegenden Erfindung in Übereinstimmung mit dem fachmännischen Verständnis nach dem Physiker Van-der-Waals benannte, relativ schwache, nicht-kovalente Wechselwirkungen zwischen Atomen oder Molekülen, deren Wechselwirkungsenergie für kugelförmige Teilchen mit etwa der sechsten Potenz des Abstandes abfällt, verstanden werden. Nach heutigem Verständnis lassen sich die Van-der-Waals-Kräfte in nachfolgende drei Bestandteile aufteilen:
- Die zur absoluten Temperatur indirekt proportionale Keesom-Wechselwirkung zwischen zwei Dipolen (Dipol-Dipol-Kräfte),
- Debye-Wechselwirkung zwischen einem Dipol und einem polarisierbaren Molekül (Dipol-induzierter-Dipol-Kräfte) und
- Londonsche-Dispersions-Wechselwirkung (London-Kräfte) zwischen zwei polarisierbaren Molekülen (induzierter-Dipol-induzierter-Dipol-Kräfte). Die London-Kräfte werden oft auch als Van-der-Waals-Kraft im engeren Sinne bezeichnet.

Alle Van-der-Waals-Kräfte sind im Vergleich zur kovalenten Bindung und Ionenbindung schwache Kräfte, wobei die vorgenannte Dispersions-Wechselwirkung im Allgemeinen der dominierende Bestandteil der drei vorgenannten Bestandteile der Van-der-Waals-Kräfte ist.

Die Erfindung beruht auf der überraschenden Erkenntnis, dass sich Implantate in einer Verpackung mit mikrostrukturierte Haftelementen ausreichend anhaften oder fixieren lassen, so dass insbesondere ein transportbedingter Abrieb zwischen Implantat und Verpackung und mithin eine Partikelkontamination des Implantats signifikant reduziert oder sogar vollständig vermieden werden kann. Dadurch kann im Falle von Implantaten als Verpackungsgut das Risiko des Auftretens von postoperativen Komplikationen signifikant verringert werden. Die Anhaftung bzw. Fixierung des Implantats an den mikrostrukturierten Haftelementen und mithin an der Innenoberfläche der Verpackung basiert dabei, wie bereits erwähnt, vorzugsweise auf Van-der-Waals-Kräften. Dadurch kann mit besonderem Vorteil das sogenannte Gecko-Prinzip wenigstens teilweise nachgeahmt werden. Ein weiterer Vorteil besteht darin, dass, abgesehen von den mikrostrukturierten Haftelementen, bevorzugt keine weiteren Haftmittel, insbesondere Klebemittel, und/oder keine mechanischen Fixiermittel zum Anhaften oder Fixieren des Implantats erforderlich sind. Die erfindungsgemäße Verpackung ist daher bevorzugt frei von weiteren Haftmitteln zum Anhaften des Implantats und/oder frei von mechanischen Fixiermitteln zum Fixieren des Implantats. Mit anderen Worten ist es bevorzugt, dass es sich bei den mikrostrukturierten Haftelementen um die einzigen Haft- und/oder Fixiermittel der erfindungsgemäßen Verpackung zum Anhaften und/oder Fixieren des Implantats handelt.

In Ausgestaltung der Erfindung weisen die mikrostrukturierten Haftelemente jeweils einen länglichen Haftelementkörper auf. Bevorzugt ist der längliche Haftelementkörper zylindrisch, insbesondere kreiszylindrisch, gestaltet. Besonders bevorzugt ist der längliche Haftelementkörper stiel- oder stengelförmig gestaltet.

Ferner weist der längliche Haftelementkörper bevorzugt eine Länge von 50 µm bis 150 µm, insbesondere 50 µm bis 130 µm, vorzugsweise 80 µm bis 100 µm, auf. Grundsätzlich können die länglichen Haftelementkörper zwar unterschiedliche Längen aufweisen. Vorzugsweise weisen die länglichen Haftelementkörper jedoch jeweils die gleiche Länge auf. Ferner können die länglichen Haftelementkörper massiv, d.h. nicht hohl, oder hohl gestaltet sein.

Ferner ist es bevorzugt, dass der längliche Haftelementkörper einen Durchmesser von 10 µm bis 40 µm, insbesondere 20 µm bis 40 µm, bevorzugt 20 µm bis 35 µm, aufweist. Unter dem Ausdruck "Durchmesser" soll im Sinne der vorliegenden Erfindung im Zusammenhang des länglichen Haftelementkörpers der mikrostrukturierten Haftelemente der größtmögliche Abstand verstanden werden, den zwei Punkte entlang einer Umfangslinie des länglichen Haftelementkörpers zueinander einnehmen können. Grundsätzlich kann der längliche Haftelementkörper einen eckenlosen, insbesondere kreisförmigen, ovalen oder elliptischen, Querschnitt oder einen eckigen, insbesondere dreieckigen, rechteckigen wie quadratischen, fünfeckigen, sechseckigen, siebeneckigen, achteckigen, neuneckigen oder zehneckigen, Querschnitt aufweisen.

In weiterer Ausgestaltung der Erfindung weisen die mikrostrukturierten Haftelemente jeweils ein freies Ende, insbesondere als Kopfteil auf dem länglichen Haftelementkörper, auf. Die Anhaftung des Implantats erfolgt vorzugsweise über das freie Ende bzw. den Kopfteil der mikrostrukturierten Haftelemente und beruht, wie bereits erwähnt, vorzugsweise überwiegend oder ausschließlich auf der Ausbildung von Van-der-Waals-Kräften. Vorzugsweise weist das freie Ende bzw. der Kopfteil einen Durchmesser auf, der größer ist als der Durchmesser des länglichen Haftelementkörpers. Mit anderen Worten weisen die mikrostrukturierten Haftelemente vorzugsweise jeweils ein gegenüber dem Durchmesser des länglichen Haftelementkörpers durchmesserverbreitertes, d.h. im Durchmesser verbreitertes, freies Ende bzw. Kopfteil auf. Dadurch ist mit besonderem Vorteil eine Optimierung der Anhaftung des Implantats auf Basis von Van-der-Waals-Kräften erzielbar. Bevorzugt weist das freie Ende bzw. der Kopfteil der mikrostrukturierten Haftelemente einen Durchmesser von 15 µm bis 70 µm, insbesondere 25 µm bis 65 µm, bevorzugt 45 µm bis 60 µm, auf. Unter dem Ausdruck "Durchmesser" soll im Sinne der vorliegenden Erfindung im Zusammenhang des freien Endes bzw. des Kopfteils der mikrostrukturierten Haftelemente der größtmögliche Abstand verstanden werden, den zwei Punkte entlang einer Umfangslinie des freien Endes bzw. des Kopfteils zueinander einnehmen können. Ferner kann das freie Ende bzw. der Kopfteil der mikrostrukturierten Haftelemente eine Höhe von 5 µm bis 20 µm, insbesondere 7 µm bis 15 µm, bevorzugt 8 µm bis 12 µm, aufweisen. Grundsätzlich kann das freie Ende bzw. der Kopfteil einen eckenlosen, insbesondere kreisförmigen, ovalen oder elliptischen, Querschnitt oder einen eckigen, insbesondere dreieckigen, rechteckigen wie quadratischen, fünfeckigen, sechseckigen, siebeneckigen, achteckigen, neuneckigen oder zehneckigen, Querschnitt aufweisen.

Ferner ist es bevorzugt, wenn sich der Durchmesser des freien Endes bzw. des Kopfteils der mikrostrukturierten Haftelemente in Richtung zu einem oberen, d.h. kopfteilnahen, Ende des länglichen Haftelementkörpers, insbesondere unter Ausbildung eines Hinterschnitts, verringert. Insbesondere können die mikrostrukturierten Haftelemente einen radialen Überstand von dem freiem Ende bzw. dem Kopfteil zu einem oberen, d.h. kopfteilnahen, Ende des länglichen Haftelementkörpers von 5 µm bis 20 µm, insbesondere 7 µm bis 15 µm, bevorzugt 8 µm bis 12 µm, aufweisen.

Ferner weisen die mikrostrukturierten Haftelemente jeweils bevorzugt ein Fußteil auf, über das der längliche Haftelementkörper eines jeweiligen mikrostrukturierten Haftelements mit der Innenoberfläche der Verpackung verbunden ist. Bevorzugt weist das Fußteil der mikrostrukturierten Haftelemente einen Durchmesser auf, der größer ist als der Durchmesser des länglichen Haftelementkörpers und/oder größer ist als der Durchmesser des freien Endes bzw. des Kopfteils der mikrostrukturierten Haftelemente. Insbesondere kann der Fußteil der mikrostrukturierten Haftelemente einen Durchmesser aufweisen, der größer ist als der Durchmesser des länglichen Haftelementkörpers und des freien Endes bzw. des Kopfteils der mikrostrukturierten Haftelemente. Beispielsweise kann der Fußteil der mikrostrukturierten Haftelemente einen Durchmesser von 15 µm bis 70 µm, insbesondere 25 µm bis 65 µm, bevorzugt 45 µm bis 60 µm, aufweisen. Unter dem Ausdruck "Durchmesser" soll im Sinne der vorliegenden Erfindung im Zusammenhang des Fußteils der mikrostrukturierten Haftelemente der größtmögliche Abstand verstanden werden, den zwei Punkte entlang einer Umfangslinie des Fußteils zueinander einnehmen können. Grundsätzlich kann das Fußteil einen eckenlosen, insbesondere kreisförmigen, ovalen oder elliptischen, Querschnitt oder einen eckigen, insbesondere dreieckigen, rechteckigen wie quadratischen, fünfeckigen, sechseckigen, siebeneckigen, achteckigen, neuneckigen oder zehneckigen, Querschnitt aufweisen.

Grundsätzlich kann das freie Ende bzw. der Kopfteil der mikrostrukturierten Haftelemente gewölbt, insbesondere konvex, gestaltet sein.

Erfindungsgemäß ist es jedoch bevorzugt, wenn das freie Ende bzw. der Kopfteil der mikrostrukturierten Haftelemente plan, d.h. eben oder nicht gewölbt, gestaltet ist. Eine derartige Ausgestaltung des freien Endes bzw. des Kopfteils ist im Hinblick auf ein Optimum bezogen auf die Ausbildung von Van-der-Waals-Kräften besonders bevorzugt.

Ferner können die mikrostrukturierten Haftelemente oder Teile davon, insbesondere der längliche Haftelementkörper der mikrostrukturierten Haftelemente, ein Aspektverhältnis, d.h. ein Längen-Durchmesser-Verhältnis (Verhältnis von Länge zu Durchmesser) von 1:3 bis 1:5 aufweisen.

Ferner können benachbarte freie Enden bzw. können Kopfteile der mikrostrukturierten Haftelemente einen gegenseitigen Abstand von 10 µm bis 60 µm, insbesondere 20 µm bis 50 µm, bevorzugt 30 µm bis 40 µm, aufweisen.

In weiterer Ausgestaltung der Erfindung weisen die mikrostrukturierten Haftelemente jeweils die Form eines Rotationshyperboloids auf. Eine solche Ausgestaltung der mikrostrukturierten Haftelemente ist besonders vorteilhaft im Hinblick auf eine zusätzliche Optimierung der Anhaftung des Implantats auf Basis von Van-der-Waals-Kräften.

In weiterer Ausgestaltung der Erfindung weisen die mikrostrukturierten Haftelemente jeweils ein spatulaartig, d.h. in Form von Einzelfasern oder -filamenten, aufgetrenntes, freies Ende, insbesondere als Kopfteil auf dem länglichen Haftelementkörper, auf. Dadurch ist ebenfalls mit besonderem Vorteil eine weitere Optimierung der Anhaftung des Implantats auf Basis von Van-der-Waals-Kräften erzielbar.

Vorzugsweise sind auf der Innenoberfläche der Verpackung wenigstens abschnittsweise wenigstens 10.000, insbesondere 15.000 bis 40.000, bevorzugt 25.000 bis 35.000, beispielsweise 29.000, mikrostrukturierte Haftelemente pro cm² Innenoberfläche der Verpackung ausgebildet. Eine solche Dichte von mikrostrukturierten Haftelementen auf der Innenoberfläche der Verpackung ist unter dem Gesichtspunkt der Erzielung optimaler Van-der-Waals-Kräfte und mithin optimaler Anhaftungsergebnisse zusätzlich von Vorteil.

In weiterer Ausgestaltung der Erfindung handelt es sich bei der Innenoberfläche der Verpackung um eine Innenoberfläche eines Verpackungsunterteils der Verpackung.

In weiterer Ausgestaltung der Erfindung handelt es sich bei der Innenoberfläche des Verpackungsunterteils um eine Oberfläche, insbesondere Bodenoberfläche, einer Vertiefung des Verpackungsunterteils. Vorzugsweise weist die Vertiefung des Verpackungsunterteils eine Form auf, welche wenigstens abschnittsweise, insbesondere nur abschnittsweise oder vollständig, zu einer Form des Implantats komplementär ist. Die Vertiefung ermöglicht somit vorzugsweise eine wenigstens abschnittsweise Aufnahme des Implantats. Dadurch ist eine zusätzliche Fixierung des Implantats und somit eine weitere Senkung des Risikos eines insbesondere transportbedingten Abriebs zwischen Verpackung und Implantat erzielbar.

In weiterer Ausgestaltung der Erfindung sind die mikrostrukturierten Haftelemente nur auf der Oberfläche, vorzugsweise nur auf der Bodenoberfläche, der Vertiefung des Verpackungsunterteils ausgebildet. Dadurch ist eine Anhaftung des Implantats nur auf der Oberfläche, insbesondere nur auf der Bodenoberfläche, der Vertiefung des Verpackungsunterteils realisierbar. Dies wiederum ermöglicht in vorteilhafterweise eine besonders geringe Kontaktfläche zwischen der Verpackung und dem Implantat und mithin eine besonders effektive Risikominimierung eines insbesondere transportbedingten Partikelabriebs zwischen Verpackung und Implantat.

In weiterer Ausgestaltung der Erfindung ist die Oberfläche, insbesondere Bodenoberfläche, der Vertiefung des Verpackungsunterteils wenigstens abschnittsweise, insbesondere nur abschnittsweise oder durchgehend, als Oberfläche einer Folie gestaltet, auf der die mikrostrukturierten Haftelemente, insbesondere abstehenden, mikrostrukturierten Haftelemente, ausgebildet sind. Mit anderen Worten ist die Vertiefung, insbesondere der Boden der Vertiefung, des Verpackungsunterteils in weiterer Ausgestaltung der Erfindung wenigstens abschnittsweise, insbesondere nur abschnittsweise oder durchgehend, mit einer Folie, auf der die mikrostrukturierten Haftelemente, insbesondere abstehenden, mikrostrukturierten Haftelemente, ausgebildet sind, beschichtet. Besonders bevorzugt ist nur die Bodenoberfläche der Vertiefung des Verpackungsunterteils als Oberfläche einer Folie, auf der die mikrostrukturierten Haftelemente, insbesondere abstehenden, mikrostrukturierten Haftelemente, ausgebildet sind, gestaltet. Die Folie ist vorzugsweise stoffschlüssig mit der Vertiefung, insbesondere dem Boden der Vertiefung, des Verpackungsunterteils verbunden, beispielsweise durch Kleben oder Kaschieren. Vorzugsweise handelt es sich bei der Folie um eine Kunststofffolie. Unter dem Ausdruck "Kunststofffolie" soll im Sinne der vorliegenden Erfindung eine Folie verstanden werden, welche ein Kunststoffmaterial aufweist oder aus einem Kunststoffmaterial besteht. Die in diesem Absatz beschriebenen Ausführungsformen der Erfindung haben den Vorteil, dass Materialien, insbesondere Kunststoffmaterialien, für die Folie verwendet werden können, die die Ausbildung von unter Anhaftungsgesichtspunkten, insbesondere auf Basis von Van-der-Waals-Kräften, besonders vorteilhaften mikrostrukturierten Haftelementen erlauben. Auf entsprechend geeignete Kunststoffmaterialien wird im Folgenden noch näher eingegangen werden.

Ferner kann die Folie, insbesondere Kunststofffolie, eine Dicke von 50 µm bis 2500 µm, insbesondere 200 µm bis 1000 µm, bevorzugt 250 µm bis 400 µm, aufweisen.

In weiterer Ausgestaltung der Erfindung weist die Folie, insbesondere Kunststofffolie, ein Kunststoffmaterial auf oder besteht aus einem Kunststoffmaterial. Bei dem Kunststoffmaterial kann es sich dabei insbesondere um ein thixotropes Kunststoffmaterial handeln. Bevorzugt ist das Kunststoffmaterial ausgewählt aus der Gruppe bestehend aus anorganische Elastomere, organische Elastomere, Siloxane, insbesondere Polyvinylsiloxan, additionsvernetzende Silikon-Elastomere, Kautschukmaterialien, Naturkautschuk, Synthesekautschuk, Styrol-Butadien-Kautschuk, Chloropren-Kautschuk, Ethylen-Propylen-Dien-Kautschuk, Polyacrylate und Mischungen davon.

In weiterer Ausgestaltung der Erfindung ist das Implantat in der Verpackung enthalten und haftet an den mikrostrukturierten, insbesondere abstehenden, mikrostrukturierten, Haftelementen an.

In weiterer Ausgestaltung der Erfindung weist die Verpackung ferner ein Verpackungsoberteil, vorzugweise ein gehäuseförmiges Verpackungsoberteil, auf. Vorzugsweise weist das Verpackungsoberteil einen rechteckförmigen, insbesondere quadratförmigen, oder halbkreisförmigen Querschnitt auf. Dadurch ist eine weitere Reduzierung der Kontaktfläche zwischen der Verpackung und dem Implantat und mithin eine weitere Minimierung des Risikos eines insbesondere transportbedingten Abriebs zwischen Verpackung und Implantat erzielbar.

Ferner kann das Verpackungsunterteil einen rechteckförmigen, insbesondere quadratförmigen, oder halbkreisförmigen Querschnitt aufweisen.

Ferner ist es bevorzugt, dass die Verpackung, insbesondere das Verpackungsunterteil und/oder Verpackungsoberteil, Hartplastik aufweist, insbesondere aufweisen, oder, insbesondere abgesehen von der oben erwähnten Folie, insbesondere Kunststofffolie, aus Hartplastik besteht, insbesondere bestehen. Das Hartplastik kann insbesondere ausgewählt sein aus der Gruppe bestehend aus Polyolefine, Polyethylen, Polypropylen, Polyetheretherketon, Polyester, Polyamide und Mischungen davon.

Beispielsweise kann es sich bei dem Implantat um ein Tibia-Implantat, d.h. um ein Implantat, das in einen Unterschenkelknochen (Tibia) implantiert wird, handeln. Das Tibia-Implantat kann hierzu mit einem Schaft und insbesondere seitlichen Finnen versehen sein, um eine benötigte Stabilität zu erzeugen.

Gemäß einem zweiten Aspekt betrifft die Erfindung ein Verfahren zum Herstellen einer Verpackung gemäß erstem Erfindungsaspekt. Das Verfahren weist folgenden Schritt auf:
a) Ausbilden oder Erzeugen von mikrostrukturierten Haftelementen, insbesondere abstehenden, mikrostrukturierten Haftelementen, auf einer Innenoberfläche einer Verpackung oder auf einer als Innenoberfläche einer Verpackung oder als Teil einer Innenoberfläche einer Verpackung vorgesehenen Folie, insbesondere Kunststofffolie.

Bevorzugt werden die mikrostrukturierten Haftelemente beim Durchführen von Schritt a) auf der Innenoberfläche eines Verpackungsunterteils, insbesondere auf der Oberfläche, bevorzugt Bodenoberfläche, einer Vertiefung eines Verpackungsunterteils, ausgebildet oder erzeugt. Das Ausbilden oder Erzeugen der mikrostrukturierten Haftelemente kann beispielsweise mittels eines Ätzverfahrens ("nanoscale sculpturing") oder Laserverfahrens durchgeführt werden.

Ferner kann das Verfahren bevorzugt einen weiteren Schritt b) Beschichten einer Oberfläche, insbesondere Bodenoberfläche, einer Vertiefung eines Verpackungsunterteils der Verpackung mit der Folie und/oder Verpacken des Implantats in/mittels der Verpackung umfassen. Das Beschichten der Oberfläche, insbesondere Bodenoberfläche, der Vertiefung des Verpackungsunterteils kann mittels einer stoffschlüssigen Fügetechnik, wie beispielsweise Kleben oder Kaschieren, erfolgen.

Bezüglich weiterer Merkmale und Vorteile des Verfahrens, insbesondere in Bezug auf die Verpackung, die mikrostrukturierten Haftelemente, die Folie sowie das Implantat, wird vollständig auf die im Rahmen des ersten Erfindungsaspekts gemachten Ausführungen Bezug genommen. Die dort insoweit beschriebenen Merkmale und Vorteile gelten sinngemäß auch für das Verfahren gemäß zweitem Erfindungsaspekt.

Gemäß einem dritten Aspekt betrifft die Erfindung die Verwendung einer Verpackung gemäß erstem Erfindungsaspekt zum Verpacken eines Implantats, wie beispielsweise Tibia-Implantats.

Bezüglich weiterer Merkmale und Vorteile der Verpackung sowie des Implantats wird vollständig auf die im Rahmen des ersten Erfindungsaspekts gemachten Ausführungen Bezug genommen. Die dort insoweit beschriebenen Merkmale und Vorteile gelten sinngemäß auch für die Verwendung gemäß drittem Erfindungsaspekt.

Gemäß einem vierten Aspekt betrifft die Erfindung ein medizinisches Kit, das eine Verpackung gemäß erstem Erfindungsaspekt sowie ein Implantat aufweist. Das Implantat ist vorzugsweise in der Verpackung enthalten und haftet vorzugsweise an den mikrostrukturierten Haftelementen, insbesondere an den abstehenden, mikrostrukturierten Haftelementen, der Innenoberfläche der Verpackung an.

Bezüglich weiterer Merkmale und Vorteile des Kits, insbesondere in Bezug auf die Verpackung sowie das Implantat, wird vollständig auf die im Rahmen des ersten Erfindungsaspekts gemachten Ausführungen Bezug genommen. Die dort insoweit beschriebenen Merkmale und Vorteile gelten sinngemäß auch für das medizinische Kit gemäß viertem Erfindungsaspekt.

Weitere Merkmale und Vorteile der Erfindung ergeben sich aus den Ansprüchen sowie aus der nachfolgenden Beschreibung eines bevorzugten Ausführungsbeispiels der Erfindung, dass anhand der Zeichnungen dargestellt ist.

### FIGURENKURZBESCHREIBUNGEN

- Fig. 1: zeigt eine schematische Perspektivdarstellung einer Ausführungsform einer erfindungsgemäßen Verpackung,
- Fig. 2: zeigt eine schematische Explosivdarstellung der Ausführungsform einer erfindungsgemäßen Verpackung nach Fig. 1 und
- Fig. 3: zeigt schematisch eine vergrößerte Detaildarstellung einer Oberfläche einer Vertiefung eines Verpackungsunterteils einer erfindungsgemäßen Verpackung in einem Bereich A nach Fig. 2.

### AUSFÜHRLICHE FIGURENBESCHREIBUNGEN

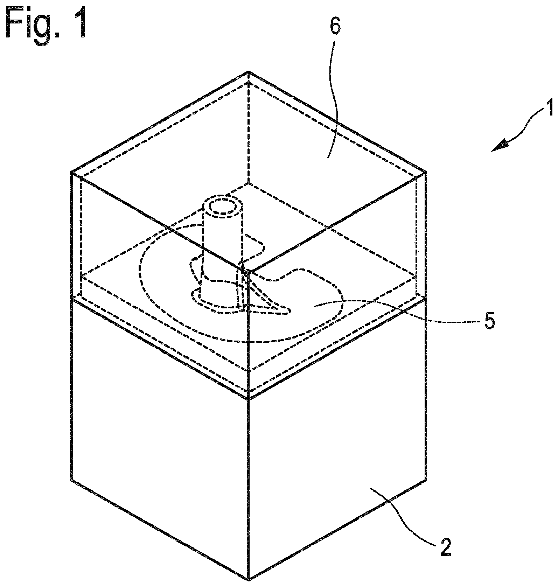

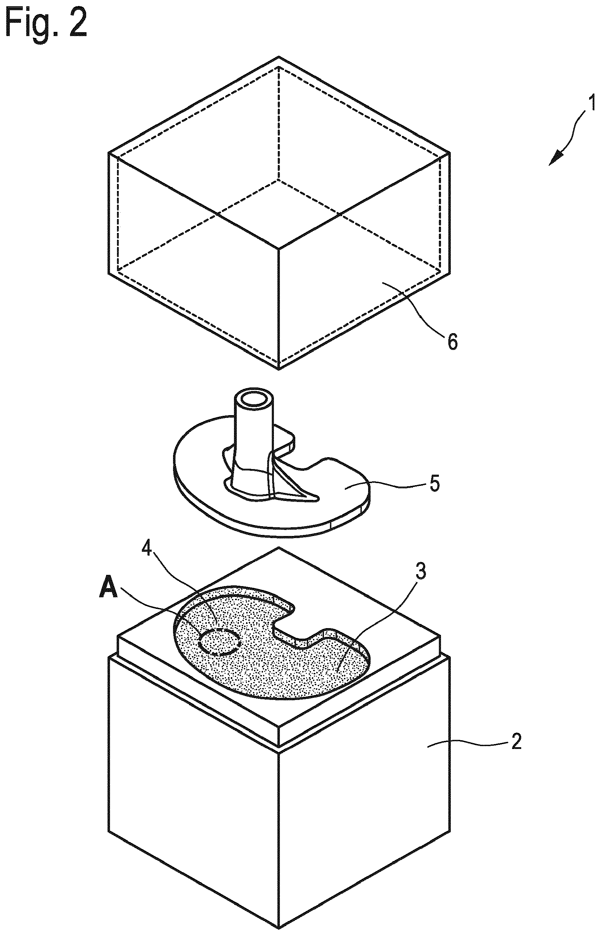

Die Fig. 1 und 2 zeigen eine Ausführungsform einer erfindungsgemäßen Verpackung 1.

Die Verpackung 1 weist ein Verpackungsunterteil 2, ein Implantat, wie beispielsweise Tibia-Implantat, 5 sowie ein Verpackungsoberteil 6 auf. Das Verpackungsunterteil 2 und das Verpackungsoberteil 6 können beispielsweise durch Verschlüsse, Rastnasen, Passungen, Presspassungen, Siegelungen oder Klebstoffe miteinander verbunden sein.

Das Verpackungsunterteil 2 weist eine Vertiefung 3 auf. Die Vertiefung 3 ist wenigstens abschnittsweise komplementär zu einer Form des Implantats 5 gestaltet. Dadurch ist eine wenigstens abschnittsweise Aufnahme des Implantats 5 in die Vertiefung 3 möglich.

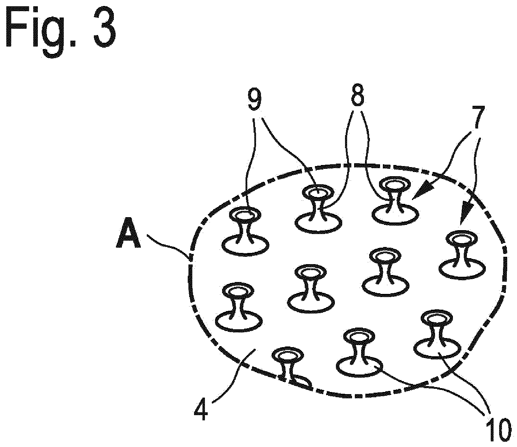

Die Oberfläche, insbesondere nur die Bodenoberfläche, 4 der Vertiefung 3 weist abstehende, mikrostrukturierte Haftelemente 7 auf (siehe Fig. 3). Dadurch ist mit besonderem Vorteil eine Anhaftung des Implantats 5 an/in der Vertiefung 3 des Verpackungsunterteils 2 realisierbar, insbesondere ohne zusätzliche Klebe- und/oder mechanische Fixiermittel. Die Anhaftung des Implantats 5 basiert vorzugsweise, insbesondere überwiegend oder ausschließlich, auf der Ausbildung von Van-der-Waals-Kräften ("Gecko-Prinzip).

Durch eine solche Anhaftung des Implantats 5 kann insbesondere ein transportbedingter Abrieb zwischen der Verpackung 1 und dem Implantat 5 reduziert oder sogar vollständig vermieden werden. Hierdurch kann beispielsweise das Risiko von postoperativen Komplikationen signifikant gesenkt werden.

Bevorzugt ist die Oberfläche, insbesondere nur die Bodenoberfläche, 4 der Vertiefung 3 als Oberfläche einer Kunststofffolie gestaltet, auf der die abstehenden, mikrostrukturierten Haftelemente 7 ausgebildet sind.

Alternativ können die abstehenden, mikrostrukturierten Haftelemente 7 durch Ätzverfahren, wie beispielsweise "nanoscale sculpturing" oder Laserverfahren auf der Oberfläche, insbesondere nur auf der Bodenoberfläche, 4 der Vertiefung 3 ausgebildet sein.

Die Kunststofffolie kann insbesondere Siloxan, insbesondere Polyvinylsiloxan, aufweisen oder aus Siloxan, insbesondere Polyvinylsiloxan, bestehen. Ferner kann die Kunststofffolie beispielsweise eine Dicke von 0,5 mm bis 2,5 mm aufweisen.

Sowohl das Verpackungsunterteil 2 als auch das Verpackungsoberteil 6 können jeweils Hartplastik aufweisen oder, insbesondere abgesehen von der vorgenannten Kunststofffolie, aus Hartplastik gefertigt sein. Das Hartplastik kann beispielsweise ausgewählt sein aus der Gruppe bestehend aus Polyolefine, Polyethylen, Polypropylen, Polyetheretherketon, Polyester, Polyamide und Mischungen davon.

Bevorzugt weist das Verpackungsoberteil 6 ferner eine Gehäuseform auf. Dadurch lässt sich mit besonderem Vorteil die Kontaktfläche zwischen dem Implantat 5 und der Verpackung 1 zusätzlich reduzieren. Dies wiederrum führt zu einer zusätzlichen Risikominimierung eines insbesondere transportbedingten Partikelabriebs zwischen der Verpackung 1 und dem Implantat 5.

Fig. 3 zeigt schematisch eine vergrößerte Detaildarstellung der Oberfläche, insbesondere Bodenoberfläche, 4 der Vertiefung 3 des Verpackungsunterteils 2.

Wie bereits erwähnt, weist die Oberfläche, insbesondere Bodenoberfläche, 4 der Vertiefung 3 des Verpackungsunterteils 2 abstehende, mikrostrukturierte Haftelemente 7 auf. Die mikrostrukturierten Haftelemente 7 weisen jeweils einen länglichen Haftelementkörper 8, ein als Kopfteil auf dem länglichen Haftelementkörper 8 gestaltetes freies Ende 9 sowie ein Fußteil 10, über das der längliche Haftelementkörper 8 mit der Oberfläche, insbesondere Bodenoberfläche, 4 der Vertiefung 3 verbunden ist, auf.

Der längliche Haftelementkörper 8 kann insbesondere eine Länge von 50 µm bis 150 µm und einen Durchmesser von 10 µm bis 40 µm aufweisen.

Das freie Ende bzw. der Kopfteil 9 der mikrostrukturierten Haftelemente 7 ist vorzugsweise gegenüber dem Durchmesser des länglichen Haftelementkörpers 8 im Durchmesser verbreitert gestaltet. Bevorzugt weist das freie Ende bzw. der Kopfteil 9 einen Durchmesser von 15 µm bis 70 µm auf. Besonders bevorzugt ist die Oberseite des freien Endes bzw. des Kopfteils 9 plan oder nur geringfügig konvex gestaltet. Ferner kann das freie Ende bzw. das Kopfteil 9 eine Höhe von 8 µm bis 12 µm, beispielsweise von 10 µm, aufweisen. Die in diesem Absatz beispielhaft beschriebenen Ausgestaltungen des freien Endes bzw. des Kopfteils 9 sind unter Anhaftungsgesichtspunkten besonders vorteilhaft.

Ferner kann es bevorzugt sein, wenn die mikrostrukturierten Haftelemente 7 in einem Bereich zwischen dem freien Ende bzw. dem Kopfteil 9 und einem oberen, d.h. kopfteilnahen, Ende des länglichen Haftelementkörpers 8, einen Hinterschnitt aufweisen. Insbesondere können die mikrostrukturierten Haftelemente 7 einen radialen Überstand von dem freiem Ende bzw. dem Kopfteil 9 zu einem oberen, d.h. kopfteilnahen, Ende des länglichen Haftelementkörpers 8 von 8 µm bis 12 µm, beispielsweise von 10 µm, aufweisen.

Vorzugsweise sind auf der Oberfläche, insbesondere Bodenoberfläche, 4 der Vertiefung 3 wenigstens abschnittsweise wenigstens 10.000, insbesondere 15.000 bis 40.000, mikrostrukturierte Haftelemente 7 pro cm² Oberfläche, insbesondere Bodenoberfläche, 4 der Vertiefung 3 ausgebildet.

Die mikrostrukturierten Haftelemente 7 können jeweils insbesondere als Rotationshyperboloid gestaltet sein.

Alternativ können die mikrostrukturierten Haftelemente jeweils an ihrem freien Ende bzw. ihrem Kopfteil 9 in Form von Einzelfasern oder -filamenten aufgetrennt sein.

Die vorstehend beschriebenen mikrostrukturierten Haftelemente 7 auf der Oberfläche, insbesondere Bodenoberfläche, 4 der Vertiefung 3 des Verpackungsunterteils 2 sind besonders vorteilhaft im Hinblick auf eine Anhaftung des Implantats 5 zum Zwecke der Reduzierung oder Vermeidung eines insbesondere transportbedingten Partikelabriebs zwischen der Verpackung 1 und dem Implantat 5. Die Anhaftung des Implantats 5 an/in der Vertiefung 3 erfolgt vorzugsweise über die freien Enden bzw. Kopfteile 9 der mikrostrukturierten Haftelemente 7 und basiert vorzugsweise überwiegend oder vollständig auf der Ausbildung von Van-der-Waals-Kräften.

## Patentansprüche

1. Verpackung (1) für ein Implantat (5), **dadurch gekennzeichnet, dass** eine Innenoberfläche (4) der Verpackung (1) wenigstens abschnittsweise abstehende, mikrostrukturierte Haftelemente (7) zum Anhaften des Implantats (5) aufweist, wobei die mikrostrukturierten Haftelemente derart gestaltet sind, dass sie ein Anhaften oder Fixieren des optional in der Verpackung enthaltenen Implantats an die Innenoberfläche der Verpackung überwiegend oder ausschließlich auf Basis von Van-der-Waals-Kräften ermöglichen.

2. Verpackung (1) nach Anspruch 1, **dadurch gekennzeichnet, dass** die mikrostrukturierten Haftelemente (7) jeweils einen länglichen Haftelementkörper (9), insbesondere mit einer Länge von 50 µm bis 150 µm und einem Durchmesser von 10 µm bis 40 µm, aufweisen.

3. Verpackung (1) nach Anspruch 2, **dadurch gekennzeichnet, dass** die mikrostrukturierten Haftelemente (7) jeweils ein freies Ende (9), insbesondere als Kopfteil auf dem länglichen Haftelementkörper (8), mit einem Durchmesser aufweisen, der größer ist als der Durchmesser des länglichen Haftelementkörpers (8), wobei das freie Ende (9) der mikrostrukturierten Haftelemente (7) vorzugsweise einen Durchmesser von 15 µm bis 70 µm aufweist.

4. Verpackung (1) nach einem der vorhergehenden Ansprüche, **dadurch gekennzeichnet, dass** die mikrostrukturierten Haftelemente (7) jeweils die Form eines Rotationshyperboloids aufweisen.

5. Verpackung (1) nach Anspruch 1, **dadurch gekennzeichnet, dass** die mikrostrukturierten Haftelemente (7) jeweils ein spatulaartig aufgetrenntes, freies Ende (9), insbesondere als Kopfteil auf dem länglichen Haftelementkörper (8), aufweisen.

6. Verpackung (1) nach einem der vorhergehenden Ansprüche, **dadurch gekennzeichnet, dass** es sich bei der Innenoberfläche (4) der Verpackung (1) um eine Innenoberfläche eines Verpackungsunterteils (2) der Verpackung (1) handelt.

7. Verpackung (1) nach Anspruch 6, **dadurch gekennzeichnet, dass** es sich bei der Innenoberfläche (4) des Verpackungsunterteils (2) um eine Oberfläche, insbesondere um eine Bodenoberfläche, einer Vertiefung (3) des Verpackungsunterteils (2) handelt, wobei die Vertiefung (3) eine Form aufweist, welche wenigstens abschnittsweise zu einer Form eines zu verpackenden Implantats (5) komplementär ist.

8. Verpackung (1) nach Anspruch 7, **dadurch gekennzeichnet, dass** die mikrostrukturierten Haftelemente (7) nur auf der Oberfläche, vorzugsweise nur auf der Bodenoberfläche, (4) der Vertiefung (3) des Verpackungsunterteils (2) ausgebildet sind.

9. Verpackung (1) nach Anspruch 7 oder 8, **dadurch gekennzeichnet, dass** die Oberfläche, insbesondere Bodenoberfläche, (4) der Vertiefung (3) des Verpackungsunterteils (2) wenigstens abschnittsweise als Oberfläche einer Folie, insbesondere einer Kunststofffolie, auf der die abstehenden, mikrostrukturierten Haftelemente ausgebildet sind, gestaltet ist.

10. Verpackung (1) nach Anspruch 9, **dadurch gekennzeichnet, dass** die Folie ein Kunststoffmaterial aufweist oder aus einem Kunststoffmaterial besteht, das ausgewählt ist aus der Gruppe bestehend aus anorganische Elastomere, organische Elastomere, Siloxane, insbesondere Polyvinylsiloxan, additionsvernetzende Silikon-Elastomere, Kautschukmaterialien, Naturkautschuk, Synthesekautschuk, Styrol-Butadien-Kautschuk, Chloropren-Kautschuk, Ethylen-Propylen-Dien-Kautschuk, Polyacrylate und Mischungen davon.

11. Verpackung (1) nach einem der vorhergehenden Ansprüche, **dadurch gekennzeichnet, dass** die Verpackung (1) ferner ein gehäuseförmiges Verpackungsoberteil (6), insbesondere mit rechteckförmigem, insbesondere quadratförmigem, oder halbkreisförmigem Querschnitt, aufweist.

12. Verpackung (1) nach einem der vorhergehenden Ansprüche, **dadurch gekennzeichnet, dass** das Implantat (5) in der Verpackung (1) enthalten ist und an den abstehenden, mikrostrukturierten Haftelementen (7) anhaftet.

13. Verfahren zum Herstellen einer Verpackung (1) nach einem der vorhergehenden Ansprüche, aufweisend den Schritt:
a) Erzeugen von mikrostrukturierten Haftelementen (7) auf einer Innenoberfläche (4) einer Verpackung (1) oder auf einer als Innenoberfläche (4) oder Teil einer Innenoberfläche (4) einer Verpackung (1) vorgesehenen Folie, insbesondere Kunststofffolie.

14. Verwendung einer Verpackung (1) nach einem der Ansprüche 1 bis 12 zum Verpacken eines Implantats.

15. Medizinisches Kit, aufweisend eine Verpackung (1) nach einem der Ansprüche 1 bis 12 und ein Implantat (5), **dadurch gekennzeichnet, dass** das Implantat (5) in der Verpackung (1) enthalten ist und an den mikrostrukturierten Haftelementen (7) der Innenoberfläche (4) der Verpackung (1) anhaftet.

## Claims

1. Packaging (1) for an implant (5), **characterized in that** an inner surface (4) of the packaging (1) has at least in sections protruding microstructured adhesive elements (7) for adhering to the implant (5), the microstructured adhesive elements being designed in such a way that they allow the implant optionally contained in the packaging to adhere or be fastened to the inner surface of the packaging mainly or exclusively on the basis of van der Waals forces.

2. Packaging (1) according to Claim **1, characterized in that** the microstructured adhesive elements (7) each have an elongated adhesive element body (9), in particular having a length of 50 µm to 150 µm and a diameter of 10 µm to 40 µm.

3. Packaging (1) according to Claim 2, **characterized in that** the microstructured adhesive elements (7) each have a free end (9), in particular as a head part on the elongated adhesive element body (8), having a diameter greater than the diameter of the elongated adhesive element body (8), the free end (9) of the microstructured adhesive elements (7) preferably having a diameter of 15 µm to 70 µm.

4. Packaging (1) according to any of the preceding claims, **characterized in that** the microstructured adhesive elements (7) each have the shape of a rotational hyperboloid.

5. Packaging (1) according to Claim 1, **characterized in that** the microstructured adhesive elements (7) each have a free end (9) which is separated in the manner of a spatula, in particular as a head part on the elongated adhesive element body (8).

6. Packaging (1) according to any of the preceding claims, **characterized in that** the inner surface (4) of the packaging (1) is an inner surface of a lower packaging part (2) of the packaging (1).

7. Packaging (1) according to Claim **6, characterized in that** that the inner surface (4) of the lower packaging part (2) is a surface, in particular a bottom surface, of a recess (3) in the lower packaging part (2), the recess (3) having a shape which is complementary at least in sections to a shape of an implant (5) to be packaged.

8. Packaging (1) according to Claim 7, **characterized in that** the microstructured adhesive elements (7) are formed only on the surface, preferably only on the bottom surface, (4) of the recess (3) in the lower packaging part (2).

9. Packaging (1) according to Claim 7 or 8, **characterized in that** the surface, in particular bottom surface, (4) of the recess (3) in the lower packaging part (2) is designed at least in sections as the surface of a film, in particular a plastic film, on which the protruding, microstructured adhesive elements are formed.

10. Packaging (1) according to Claim 9, **characterized in that** the film comprises a plastic material or consists of a plastic material which is selected from the group consisting of inorganic elastomers, organic elastomers, siloxanes, especially polyvinylsiloxane, additioncrosslinking silicone elastomers, rubber materials, natural rubber, synthetic rubber, styrene-butadiene rubber, chloroprene rubber, ethylene-propylene-diene rubber, polyacrylates and mixtures thereof.

11. Packaging (1) according to any of the preceding claims, **characterized in that** the packaging (1) further has a housing-shaped upper packaging part (6), in particular having a rectangular, especially square, or semicircular cross-section.

12. Packaging (1) according to any of the preceding claims, **characterized in that** the implant (5) is contained in the packaging (1) and adheres to the protruding, microstructured adhesive elements (7).

13. Method for producing a packaging (1) according to any of the preceding claims, comprising the step of:
a) generating microstructured adhesive elements (7) on an inner surface (4) of a packaging (1) or on a film, especially plastic film, provided as an inner surface (4) or part of an inner surface (4) of a packaging (1).

14. Use of a packaging (1) according to any of Claims 1 to 12 for packaging an implant.

15. Medical kit, comprising a packaging (1) according to any of Claims 1 to 12 and an implant (5), **characterized in that** the implant (5) is contained in the packaging (1) and adheres to the microstructured adhesive elements (7) of the inner surface (4) of the packaging (1).

## Revendications

1. Emballage (1) pour un implant (5), **caractérisé en ce qu'**une surface intérieure (4) de l'emballage (1) présente au moins par sections des éléments adhésifs microstructurés (7) en saillie pour faire adhérer l'implant (5), les éléments adhésifs microstructurés étant conçus de telle sorte qu'ils permettent une adhérence ou une fixation de l'implant éventuellement contenu dans l'emballage à la surface intérieure de l'emballage principalement ou exclusivement sur la base de forces de Van der Waals.

2. Emballage (1) selon la revendication 1, **caractérisé en ce que** les éléments adhésifs microstructurés (7) présentent chacun un corps d'élément adhésif allongé (9), notamment d'une longueur de 50 µm à 150 µm et d'un diamètre de 10 µm à 40 µm.

3. Emballage (1) selon la revendication 2, **caractérisé en ce que** les éléments adhésifs microstructurés (7) présentent chacun une extrémité libre (9), notamment en tant que partie de tête sur le corps d'élément adhésif allongé (8), d'un diamètre supérieur au diamètre du corps d'élément adhésif allongé (8), l'extrémité libre (9) des éléments adhésifs microstructurés (7) présentant de préférence un diamètre de 15 µm à 70 µm.

4. Emballage (1) selon l'une quelconque des revendications précédentes, **caractérisé en ce que** les éléments adhésifs microstructurés (7) présentent chacun la forme d'un hyperboloïde de révolution.

5. Emballage (1) selon la revendication 1, **caractérisé en ce que** les éléments adhésifs microstructurés (7) présentent chacun une extrémité libre (9) séparée à la manière d'une spatule, notamment en tant que partie de tête sur le corps d'élément adhésif allongé (8).

6. Emballage (1) selon l'une quelconque des revendications précédentes, **caractérisé en ce que** la surface intérieure (4) de l'emballage (1) consiste en une surface intérieure d'une partie inférieure d'emballage (2) de l'emballage (1).

7. Emballage (1) selon la revendication 6, **caractérisé en ce que** la surface intérieure (4) de la partie inférieure d'emballage (2) consiste en une surface, notamment en une surface de fond, d'une cavité (3) de la partie inférieure d'emballage (2), la cavité (3) présentant une forme qui est complémentaire, au moins par sections, d'une forme d'un implant (5) à emballer.

8. Emballage (1) selon la revendication 7, **caractérisé en ce que** les éléments adhésifs microstructurés (7) sont réalisés uniquement sur la surface, de préférence uniquement sur la surface de fond, (4) de la cavité (3) de la partie inférieure d'emballage (2).

9. Emballage (1) selon la revendication 7 ou 8, **caractérisé en ce que** la surface, notamment la surface du fond, (4) de la cavité (3) de la partie inférieure d'emballage (2) est conçue au moins par sections en tant que surface d'un film, notamment d'un film plastique, sur laquelle sont réalisés les éléments adhésifs microstructurés en saillie.

10. Emballage (1) selon la revendication 9, **caractérisé en ce que** le film présente un matériau plastique ou est constitué d'un matériau plastique choisi dans le groupe constitué par les élastomères inorganiques, les élastomères organiques, les siloxanes, notamment le polyvinylsiloxane, les élastomères de silicone réticulant par addition, les matériaux en caoutchouc, le caoutchouc naturel, le caoutchouc synthétique, le caoutchouc de styrène-butadiène, le caoutchouc de chloroprène, le caoutchouc d'éthylène-propylène-diène, les polyacrylates et leurs mélanges.

11. Emballage (1) selon l'une quelconque des revendications précédentes, **caractérisé en ce que** l'emballage (1) présente en outre une partie supérieure d'emballage (6) en forme de boîtier, notamment de section transversale rectangulaire, notamment carrée, ou semicirculaire.

12. Emballage (1) selon l'une quelconque des revendications précédentes, **caractérisé en ce que** l'implant (5) est contenu dans l'emballage (1) et adhère aux éléments adhésifs microstructurés en saillie (7).

13. Procédé de fabrication d'un emballage (1) selon l'une quelconque des revendications précédentes, présentant l'étape suivante :
a) la production d'éléments adhésifs microstructurés (7) sur une surface intérieure (4) d'un emballage (1) ou sur un film, notamment un film plastique, prévu en tant que surface intérieure (4) ou partie d'une surface intérieure (4) d'un emballage (1).

14. Utilisation d'un emballage (1) selon l'une quelconque des revendications 1 à 12 pour emballer un implant.

15. Kit médical, présentant un emballage (1) selon l'une quelconque des revendications 1 à 12 et un implant (5), **caractérisé en ce que** l'implant (5) est contenu dans l'emballage (1) et adhère aux éléments adhésifs microstructurés (7) de la surface intérieure (4) de l'emballage (1).
